# EUROPÄISCHE PATENTANMELDUNG

(11) **EP 0 614 867 A2**
(43) Veröffentlichungstag der Anmeldung: **14.09.1994**
(21) Anmeldenummer: 94103272.4
(22) Anmeldetag: 04.03.1994
(51) Int. Cl.: C07C 43/13, H01L 39/24

(54) **Flüchtige Metall-alkoholate bifunktioneller beta-Etheralkohole, Verfahren zu deren Herstellung und ihre Verwendung**

(30) Priorität: 11.03.1993 DE 4307663
(71) Anmelder: HOECHST AKTIENGESELLSCHAFT, D-65929 Frankfurt am Main (DE)
(72) Erfinder: Herrmann, Anton Wolfgang, Prof. Dr., D-85354 Freising (DE); Huber, Norbert, DCh., D-85386 Dietersheim (DE)

(57) **Zusammenfassung**

Flüchtige Komplexverbindungen der allgemeinen Formel M[O-CR¹R²-CH₂-O-CH₃]ₙ, bei denen R¹ und R² unabhängig voneinander Methyl oder eine vollständig fluorierte Alkylgruppe mit 1 bis 9 C-Atomen, M ein Metall oder Halbmetall der Gruppen 1, 2 oder 4 bis 15 des Periodensystems der Elemente oder Uran oder Thorium und die Wertigkeit n des Metalls oder Halbmetalls 1, 2, 3 oder 4 beträgt, werden hergestellt, indem man β-Etheralkohole der allgemeinen Formel HO-CR¹R²-CH₂OCH₃ mit homoleptischen Metall-Amiden in einem aprotischen organischen Lösungsmittel umsetzt. Die Erfindung betrifft auch die Verwendung der Komplexverbindungen als molekulare Vorstufen zur Gasphasen-Abscheidung von Metallen und Metalloxiden.

## Beschreibung

Die vorliegende Erfindung betrifft die Herstellung von homoleptischen Metall-alkoholaten hoher Flüchtigkeit.

Unter der Bezeichnung homoleptische Metallkomplexe sollen im Zusammenhang mit der Erfindung solche metallorganischen Verbindungen verstanden werden, bei denen jedes Metallatom nur eine einzige Ligandensorte pro Formeleinheit enthält. Die erfindungsgemäßen Metall-alkoholate eignen sich als Vorstufen für die chemische Abscheidung von Metalloxiden aus der Gasphase ("CVD-Verfahren" = Chemical-Vapour-Deposition).

Metall-alkoxide, auch Metall-alkoholate genannt, werden in der Literatur als Ausgangsverbindungen für oxidische Materialien in CVD-Verfahren beschrieben (siehe Bradley, Chem. Rev., (1989) Bd. 89, S. 1317-1322). Allerdings wird selten darauf hingewiesen, daß die allermeisten Metall-alkoholate für CVD-Zwecke nicht flüchtig genug sind. Dieser Nachteil ist auf die für gewöhnlich oligomeren oder polymeren Molekülstrukturen der Metall-alkoholate zurückzuführen, die sich ihrerseits aus der strukturellen Brückenbildungsneigung von Alkoholatliganden sowie der Tendenz der meisten Metalle zur Ausbildung hoher Koordinationszahlen ergeben. Metallkomplexe einfacher, d.h. monofunktioneller Alkohole sind in den Wertigkeitsstufen n = 1 bis 4 fast immer oligomer und polymer und deshalb wenig flüchtig oder unflüchtig. Beispiele sind die Chrom-, Bismut-, Blei- und Zinn-Komplexe M(OR)ₙ mit R = CH₃, C₂H₅, n-C₃H₇; und M = Cr, Bi, Pb, Sn, (vgl. D.C. Bradley, A.G. Mehrotra, D.P. Gaur, "Metal Alkoxides", Academic Press, New York 1978, S. 43-74).

Zur Verbesserung der Flüchtigkeit homoleptischer Metall-alkoholate hat es nicht an Konzepten und Versuchen gefehlt. Die Flüchtigkeit ist bei Molekülverbindungen um so größer, je kleiner die Molmasse und um so geringer die intermolekularen Wechselwirkungen in der (Kristall-)Gitterpackung sind. Man hat deshalb in Metallkomplexen vom Typ M(OR)ₙ mit M = Metall, n = Wertigkeitsstufe des Metalls und R = organischer Rest den Substituenten R vergrößert, um dadurch die Tendenz zur Oligomerisierung herabzusetzen. Dieses Ziel wurde zwar in einigen Fällen erreicht, doch sind auch für den als Idealfall geltenden Substituenten R = tert.-Butyl (^{t}C₄H₉) die Flüchtigkeiten nicht groß genug, oder aber die Verbindungen sind bei den für die Sublimation erforderlichen Temperaturen nicht mehr thermodynamisch stabil. So zersetzt sich beispielsweise Cr(O^{t}C₄H₉)₃ beim Erhitzen im Vakuum in die homoleptischen Cr^{II}-und Cr^{IV}-Komplexe, von denen ersterer wiederum polymer und unflüchtig ist, d.h. im Sublimationsrückstand verbleibt, vgl. D.C. Bradley et al. (siehe oben), S. 37.

Ein weiterer häufig versuchter Ansatz besteht darin, daß man chelatisierende Alkoholat-Liganden vom Typ β-Etheralkohol und β-Aminoalkohol der Formeln -O-CH₂-CH₂-OR beziehungsweise -O-CH₂-CH₂NR₂ verwendet. Diese Liganden haben den Vorteil, daß sie im Prinzip über das Ether-Sauerstoffatom und das Amin-Stickstoffatom die Koordinationsphäre des Metallions effizienter absättigen können als einfache Alkoholate -OR. Es zeigte sich jedoch, daß entweder die monomere Konstitution ("einkernige Metall-alkoholate") und/oder die erwünschte Flüchtigkeit nicht erreicht wird (siehe: S.G. Goel et al., Inorg. Chem., Jg. 1990, Bd. 29, S. 4640 bis 4646).

Ein typisches Beispiel für das Versagen dieses Konzepts ist der in der Literatur bekannte Bismut(III)-Komplex der Formel Bi(OCH₂CH₂OCH₃)₃. Dieser Komplex läßt sich bis zu seiner Zersetzungstemperatur auch unter Hochvakuum-Bedingungen nicht in die Dampfphase überführen. Somit scheidet er für die Herstellung oxidischer Bismutverbindungen aus der Gasphase nach dem CVD-Verfahren aus. Nur Kupfer(II)-Komplexe Cu(OCH₂CH₂NR₂)₂ (R = -CH₃, -C₂H₅) sind monomer und flüchtig (vgl. S.G. Goel et al., Polyhedron, Jg. 1990, Bd. 9, S. 611-613). Aber auch von Kupfer(II) sind die β-Etheralkoholate Cu(OCH₂CH₂OR)₂ nicht flüchtig, weil sie polymere Strukturen haben, vgl. S.G. Goel, obiges Zitat.

Es wurde unlängst gezeigt, daß man zu sehr hohen Koordinationszahlen neigende Metallionen, insbesondere zweiwertiges Barium, durch mehrfunktionelle Tertiäralkohole in eine hinreichend flüchtige Form bringen kann, vgl. DE-A-42 37 522. Die dort verwendeten Alkoholatliganden sind für Metallionen in den Oxidationsstufen drei und höher allerdings nicht geeignet.

Es bestand deshalb die Aufgabe, Metall-alkoholat-komplexe mit Metallionen der Wertigkeitsstufen 1, 2, 3 und 4 zu entwickeln, die flüchtig, gleichzeitig aber thermisch hinreichend stabil sind, mit dem Ziel, diese Metallkomplexe unzersetzt in die Gasphase zu transferieren und dann aus der Gasphase entweder das freie Metall selbst oder als oxidische Schichten des Metalls auf Substraten nach dem CVD-Verfahren herzustellen.

Erfindungsgemäß wird diese Aufgabe gelöst durch flüchtige Komplexverbindungen der allgemeinen Formel M[O-CR¹R² -CH₂-O-CH₃]ₙ, wobei R¹ und R² unabhängig voneinander Methyl oder eine vollständig fluorierte Alkylgruppe mit 1 bis 9 C-Atomen, M ein Metall oder Halbmetall der Gruppen 1, 2 oder 4 bis 15 des Periodensystems der Elemente oder Uran oder Thorium und die Wertigkeit n des Metalls oder Halbmetalls 1, 2, 3 oder 4 beträgt.

Es wurde überraschend gefunden, daß die in der Komplexchemie mitunter verwendeten β-Etheralkohole, die als solche nicht zu hinreichend flüchtigen Metall-alkoholaten führen, unter der Bedingung besonders flüchtige Metallkomplexe ergeben, wenn dieser Ligandentypus an dem Kohlenstoffatom, das die Hydroxylgruppe trägt, mit Methylgruppen oder vollständig fluorierte Alkylgruppen substituiert ist und wenn die Etherfunktion am anderen Ende des Liganden eine Methylgruppe aufweist.

Im einzelnen wird die Kettenlänge der Alkylgruppen aber so aufeinander abgestimmt, daß die Flüchtigkeit der resultierenden Metall-alkoholate optimiert ist. Insbesondere sind wegen der geringstmöglichen Molmasse die Liganden HO-C(CH₃)₂-CH₂-O-CH₃ und HO-C(CF₃)₂-CH₂-O-CH₃ bevorzugt.

Den erstgenannten Liganden synthetisiert man durch die nachfolgend aufgezeigte Route ausgehend von einem handelsüblichen Epoxid. 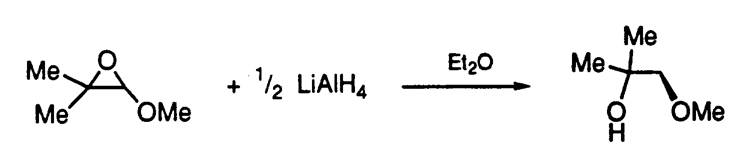
Die β-Etheralkohole lassen sich leicht zu den neuen Metall-alkoholaten umsetzen. Dazu läßt man die β-Etheralkohole, vorzugsweise mit den homoleptischen Metallamiden der allgemeinen Formel M[NR³₂]ₙ gemäß dem nachfolgend dargestellten Reaktionsmuster reagieren, wobei R³ einen einfachen Alkylrest mit 1 bis 4 C-Atomen, insbesondere Methyl, oder Di- oder Trialkylsilyl mit einer Alkylgruppe mit 1 bis 8 C-Atomen, M ein Metall oder Halbmetall aus den Gruppen 1, 2 und 4 bis 15 des Periodensystems der Elemente und n die Metallwertigkeit mit den Zahlenwerten 1, 2, 3 oder 4 darstellt. 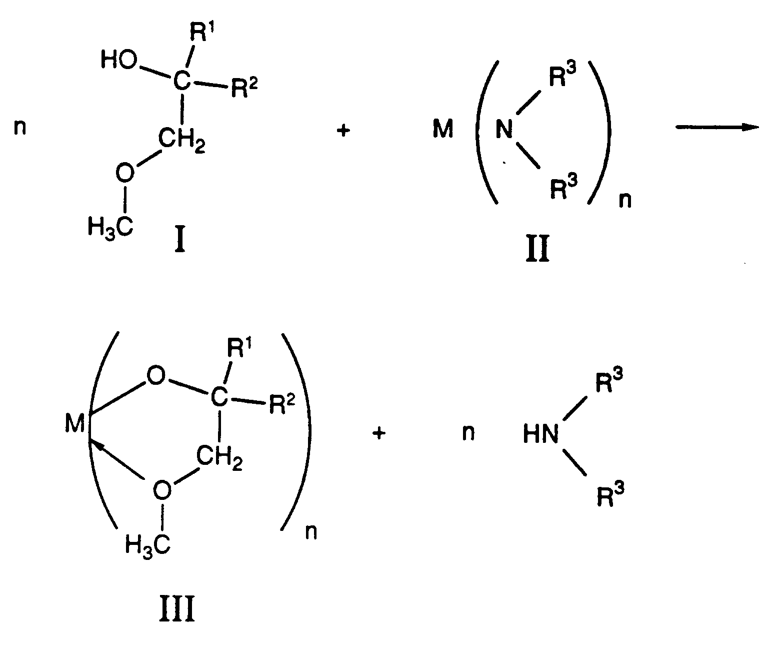
Es zeigt sich nun, daß insbesondere die richtige Wahl der Substituenten R¹ und R² zu Metallkomplexen unerwartet hoher Flüchtigkeit führt, verglichen mit den teils literaturbekannten Grundverbindungen mit R¹ = R² = H.

Die vorstehend beschriebenen β-Etheralkohole ergeben außergewöhnlich flüchtige Metall-alkoholate. Letztere eignen sich als molekulare Vorstufen zur Gasphasen-Abscheidung von Metallen oder Metalloxiden, beispielsweise für Kontaktmetallisierungsschichten und für Beschichtungen mit Oxidkeramiken.

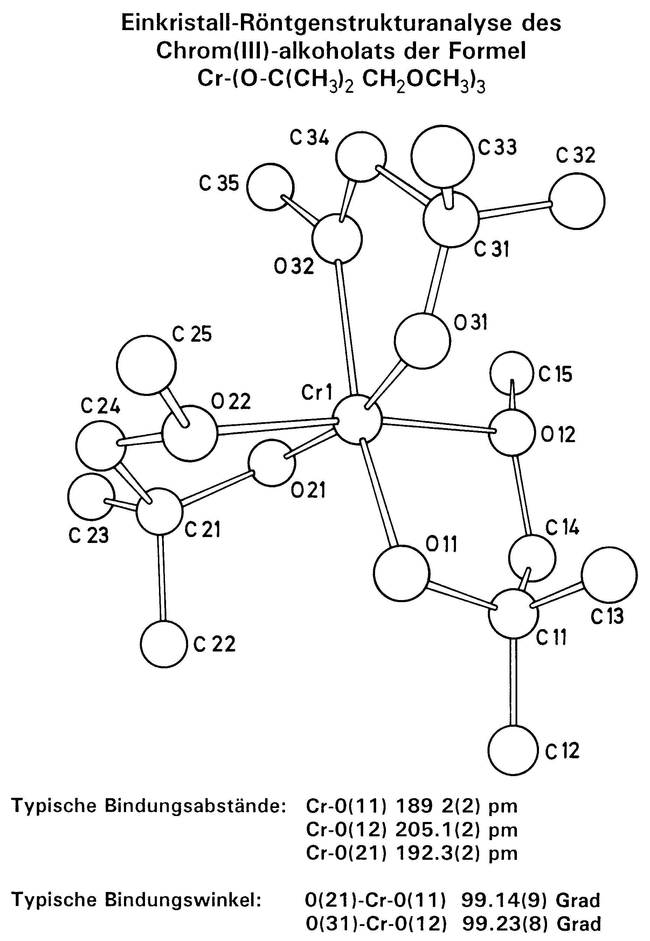

Durch die nachfolgenden Ausführungsbeispiele soll die Erfindung noch deutlicher beschrieben werden, ohne auf die konkret dargestellten Ausführungsformen beschränkt zu sein. Im Rahmen der Beispiele wurden alle Arbeiten in ausgeheizten Glasapparaturen unter absolut strengem Ausschluß von Luft und Feuchtigkeit durchgeführt (Glovebox- und Vacuumline-Techniken). Zur Herstellung der Metallamid-Vorstufen II verfährt man wie in der Literatur beschrieben, muß aber auf absolute Trockenheit der Metallhalogenid-Einsatzstoffe achten. Das Bismut(III)-amid der Formel Bi{N[Si(CH₃)₃]₂}₃ war zwar literaturbekannt, wurde aber synthesechemisch optimiert, so daß es jetzt als bequem zugängliche Startverbindung in der Bismutchemie gelten kann. Abb. 1 zeigt die monomolekulare Struktur des Chromalkoxids Cr[-OC(CH₃)₂CH₂-O-CH₃]₃ wie sie sich aus einer Einkristall-Röntgenstrukturanalyse ergibt.

Es ist überraschend, daß der einfachste β-Etheralkohol HO-CH₂CH₂-O-CH₃ mit keinem Metall monomere Chelatkomplexe bildet. Das Konzept der β-ständigen Alkoxygruppen allein löst also das Struktur/Flüchtigkeits-Problem von Alkoholat-Komplexen nicht. Auch erreicht der von Buhro et al. beschriebene (siehe W.E. Buhro et al., Inorg. Chem., Bd. 29 (1990), S. 358-360) Aminoalkoholat-Ligand -O-CH(CH₃)-CH₂N(CH₃)₂ nicht die diesbezüglich günstigen Eigenschaften der erfindungsgemäßen Liganden. So ist der Bismutkomplex Bi[-O-CH(CH₃)-CH₂N(CH₃)₂]₃ wesentlich schlechter flüchtig (90° C/10^{⁻}⁴ Torr; vergl. Buhro et al., wie oben zitiert) als der hier beschriebene Komplex (Bi[-O-C(CH₃)₂-CH₂-O-CH₃]₃ (≦ 25 ⁰C/10⁻⁴ Torr). Außerdem ist der letztgenannte Komplex wie alle anderen im Rahmen der Erfindung beschriebenen Alkoholat-Komplexe rückstandsfrei sublimierbar.

### Beispiel 1

### Herstellung von 1-Methoxy-2-methyl-2-propanol

### Route a:

Zu 0,50 mol Methylmagnesiumiodid in 170 ml Diethylether werden bei 0 °C 19,8 ml (Δ 0.20 mol) Methoxyessigsäure-methylester in 150 ml Diethylether über eine Zeitspanne von 30 min zugegeben. Nach 15 h Rühren wird der Ansatz auf 50 g Eis gegossen. Danach wird soviel gesattigte wäßrige NH₄Cl-Lösung zugegeben, bis sich gerade der ganze Niederschlag löst. Die organische Phase wird mit 30 ml gesättigter NaHCO₃-Lösung und zweimal mit Eiswasser gewaschen. Die vereinigten Wasserphasen werden mit Ether gewaschen. Danach wird die organische Phase über MgSO₄ getrocknet. Fraktionierte Destillation bei 62 bis 63 °C/100 bis 107 Torr ergibt 10,2 g 1-Methoxy-2-methyl-2-propanol (Δ 49 % Ausbeute).

### Route b (vergl. Formelschema):

Zu 55 ml (Δ 220 mmol) einer 4-molaren Lösung von Li[AIH₄] in Diethylether wird bei 0 °C 36,98 g (Δ 362 mmol) 2,2-Dimethyl-1-methoxyethylenoxid in 60 ml Diethylether über einen Zeitraum von 30 min zugetropft. Nach 20 h Rühren wird der Ansatz auf 250 g Eis gegossen. Danach wird wie bei Variante a aufgearbeitet. Fraktionierte Destillation bei 62 bis 63 °C/ 100-107 Torr ergibt 16,7 g 1-Methoxy-2-methyl-2-propanol (Δ 44 % Ausbeute).

| Die chemische Elementaranalyse ergab für die Verbindung mit der Summenformel C₅H₁₂O₂ (MG = 104,15) folgendes Ergebnis: | | |
|---|---|---|
| Berechnet: | C = 57,66 | H = 11,61 |
| Gefunden: | C = 57,58 | H = 11,48. |

### Beispiel 2

Herstellung von Tris[1-methoxy-2-methyl-2-propanolato]chrom(III): Zu einer Lösung von 0,75 g (△ 2,13 mmol) Cr[N(i-C₃H₇)₂]₃ in 40 ml n-Hexan wird bei -30 °C eine Menge von 0,67 g (△ 6,39 mmol) 1-Methoxy-2-methyl-2-propanol zugetropft. Anschließend wird über eine Zeit von 16 h bei einer Temperatur von 25 °C gerührt. Das Lösungsmittel wird im Vakuum entfernt. Das dunkelgrüne Produkt wird bei 60 bis 65 °C/ 1,5 Torr sublimiert. Daraus resultierten 0,80 g Tris[1-methoxy-2-methyl-2-propanolato]chrom(III) (Δ 97 % Ausbeute).

| Die chemische Elementaranalyse ergab für die Verbindung mit der Summenformel C₁₅H₃₃CrO₆ (MG = 361,42) folgendes Ergebnis; | | |
|---|---|---|
| Berechnet: | C = 49,85 | H = 9,20 |
| Gefunden: | C = 49,71 | H = 8,97. |

### Beispiel 3

### Tris[1-methoxy-2-methyl-2-propanolato]bismut(III)

### Variante a:

Zu einer Lösung von 2,86 g (Δ 4,14 mmol) Bi[N(i-C₃H₇)₂]₃ in 40 ml Diethylether werden bei -30 °C 1,29 g HOCMe₂CH₂OMe (Δ 12,42 mmol) zugetropft. Anschließend wird über eine Zeit von 2 d bei 25 °C gerührt. Nach Eindampfen der Reaktionslösung im Hochvakuum werden circa 5 ml n-Pentan aufkondensiert und das weiße Produkt bei -30 °C kristallisiert. Die Ausbeute beträgt 1,83 g (Δ 85 %). Die Verbindung sublimiert zügig bei 60 °C/10⁻² Torr.

### Variante b:

Zu einer Lösung von 7,93 g (Δ 43,2 mmol) Na[N(SiMe₃)₂] in 25 ml Diethylether werden bei -78 °C 5,09 g (Δ 48,8 mmol) HOCMe₂CH₂OMe über eine Zeitspanne von 15 min zugegeben. Man läßt dann 15 h bei 25 °C rühren. Die Lösungsmittel werden anschließend im Hochvakuum entfernt. Daraufhin gibt man bei -196 °C zur zähen Masse 4,38 g BiCl₃ (Δ 13,9 mmol) und 140 ml THF zu und läßt noch 20 h bei 25 °C rühren. Nach Eindampfen Im Hochvakuum wird aus dem Rückstand das weiße, mikrokristalline Produkt bei 60 °C/10⁻² Torr heraussublimiert. Die Ausbeute beträgt 5,12 g (Δ 71 %).

| Die chemische Elementaranalyse ergab für die Verbindung mit der Summenformel C₁₅H₃₃BiO₆ (MG = 518,40) folgendes Ergebnis: | | |
|---|---|---|
| Berechnet: | C = 34,75 | H = 6,42 |
| Gefunden: | C = 34,61 | H = 6,30. |

### Beispiel 4

### Bis[1-methoxy-2-methyl-2-propanolato]zinn(II)

Zu einer Lösung von 2,32 g (Δ 5,27 mmol) Sn[N(SiMe₃)₂]₂ in 30 ml THF werden bei -30 °C 1,10g HOCMe₂CH₂OMe (Δ 10.6 mmol) zugetropft. Anschließend wird über eine Zeit von 48 h bei 25 °C gerührt. Die Lösungsmittel werden im Hochvakuum entfernt. Das weiße Produkt sublimiert man bei 70 °C/1,5 Torr. Die Ausbeute beträgt 1,46 g (Δ 85 %).

| Die chemische Elementaranalyse ergab für die Verbindung mit der Summenformel C₁₀H₂₂SnO₄ (MG = 324,97) folgendes Ergebnis: | | |
|---|---|---|
| Berechnet: | C = 36,96 | H = 6,82 |
| Gefunden: | C = 36,71 | H = 6,49. |

### Beispiel 5

### Bis[1-methoxy-2-methyl-2-propanolato]blei(II)

Zu 2,27 g (Δ 4,31 mmol) Pb[N(SiMe₃)₂]₂ in 40 ml Pentan wird bei -78 °C innerhalb von 1 h 1,8 g HOCMe₂CH₂OMe (Δ 17.2 mmol) in 10 ml Pentan zugetropft. Anschließend wird über eine Zeit von 48 h bei Raumtemperatur gerührt. Das Lösungsmittel und überschüssiger Alkohol werden im Hochvakuum entfernt, etwa 5 ml Pentan aufkondensiert und das weiße Produkt bei -30 °C kristallisiert. Es läßt sich bei 60 bis 80 °C/10⁻² Torr sublimieren.

| Die chemische Elementaranalyse ergab für die Verbindung mit der Summenformel C₁₀H₂₂PbO₄ (MG = 413,47) folgendes Ergebnis: | | |
|---|---|---|
| Berechnet: | C = 29,05 | H = 5,36 |
| Gefunden: | C = 28,32 | H = 5,08. |

### Beispiel 6

### Bis[1-methoxy-2-methyl-2-propanolato]kupfer(II)

Zu 6,86 g (Δ 58,1 mmol) 1-Methoxy-2-methyl-2-propanol in 30 ml Benzol wird 1,59 g (Δ 12,7 mmol) reines in Substanz isoliertes Bis(methoxy)kupfer, hergestellt aus Kupferdichlorid und Lithiummethanolat in Methanol, zugegeben. Anschließend wird die Suspension über eine Zeit von 4 h unter Rückfluß gekocht und das entstehende Methanol während der Reaktion azeotrop abdestilliert. Verbleibendes Losungsmittel wird im Hochvakuum entfernt, wonach ein moosgrüner Rückstand von 2,86 g des Produktes Bis[1-methoxy-2-methyl-2-propanolato]Kupfer(II) (Δ 78 %) erhalten wird. Das Produkt kann bei 80 °C/10⁻² Torr sublimiert werden.

## Patentansprüche

1. Flüchtige Komplexverbindung der allgemeinen Formel M[O-CR¹R²-CH₂-O-CH₃]ₙ (III), wobei R¹ und R² unabhängig voneinander Methyl oder eine vollständig fluorierte Alkylgruppe mit 1 bis 9 C-Atomen, M ein Metall oder Halbmetall der Gruppen 1, 2 oder 4 bis 15 des Periodensystems der Elemente oder Uran oder Thorium und die Wertigkeit n des Metalls oder Halbmetalls 1, 2, 3 oder 4 beträgt.

2. Komplexverbindung gemäß Anspruch 1, dadurch gekennzeichnet, daß n = 2 und M Calcium, Blei, Kupfer oder Zinn bedeutet.

3. Komplexverbindung nach Anspruch 1, dadurch gekennzeichnet, daß n = 3 ist und M Aluminium, Thallium, Bismut, Chrom oder Eisen bedeutet.

4. Komplexverbindung gemäß Anspruch 1, dadurch gekennzeichnet, daß n = 4 ist und M Uran oder Thorium bedeutet.

5. Komplexverbindung gemäß Anspruch 1, dadurch gekennzeichnet, daß die vollständig fluorierte Alkylgruppe R¹ oder R² maximal 6 C-Atome enthält.

6. Verfahren zur Herstellung einer flüchtigen Komplexverbindung nach einem der Ansprüche 1 bis 5, dadurch gekennzeichnet, daß man β-Etheralkohole der allgemeinen Formel HO-CR¹R²-CH₂OCH₃ (I) mit homoleptischen Metall-Amiden der allgemeinen Formel M[NR³₂]ₙ in einem aprotischen organischen Lösungsmittel umsetzt, wobei R³ einen Alkylrest mit 1 bis 4 C-Atomen, insbesondere Methyl, oder Dialkylsilyl- oder Trialkylsilyl-Rest mit einer Alkylgruppe mit 1 bis 8 C-Atomen darstellt und M, R¹ und R² die in Anspruch 1 angegebene Bedeutung haben.

7. Verwendung von flüchtigen Komplexverbindungen nach einem der Ansprüche 1 bis 5, hergestellt nach Anspruch 6, als molekulare Vorstufen zur Gasphasen-Abscheidung von Metallen und Metalloxiden, beispielsweise für Beschichtungen mit Reinstmetallen, insbesondere zur Kontaktmetallisierung, oder mit Oxidkeramiken.
